# EUROPEAN PATENT SPECIFICATION

(11) **EP 0 527 939 B1**
(45) Date of publication and mention of the grant of the patent: **09.07.1997**
(21) Application number: 91910484.4
(22) Date of filing: 25.04.1991
(51) Int. Cl.: A61B 17/32, A61M 29/02, A61B 17/22, A61B 17/39

(54) **DILATATION CATHETER ASSEMBLY WITH CUTTING ELEMENT**
DILATATIONSKATHETEREINHEIT MIT SCHNEIDEELEMENT
ENSEMBLE CATHETER DE DILATATION A ELEMENT COUPANT

(30) Priority: 11.05.1990 US 522148
(43) Date of publication of application: 24.02.1993
(73) Proprietor: APPLIED MEDICAL RESOURCES, INC., Laguna Hills, CA 92653 (US)
(72) Inventor: CLAYMAN, Ralph, V., M., D., St. Louis, MO 63105 (US)
(74) Representative: Enskat, Michael Antony Frank
(86) International application number: US9102857
(87) International publication number: WO9117714

(56) References cited:
- EP-A- 0 315 730
- WO-A-89/01800
- DE-A- 2 426 781
- DE-A- 3 402 573
- FR-A- 2 594 322
- US-A- 4 273 128
- US-A- 4 709 698
- US-A- 4 799 479

## Description

The present invention relates to dilation catheter assemblies.

Dilation catheters are used to dilate body vessels, orifices and conduits such as an artery narrowed by atherosclerotic plaque and/or fibromuscular disease or to dilate a constricted or obstructed ureter or urethra. Previously proposed devices basically consist of an elongate catheter having an inflatable extensible elastomeric (rubber-like) or non-extensible variety, at or near its distal end. A guide wire or other axial support means is often included to improve the torque control or "steerability" of the apparatus.

The major advantage of dilation catheter use over conventional surgery is that it is less invasive. Nonetheless, the tissue that is stressed is often also subjected to significant trauma. As the bladder expands, it exerts pressure on the surrounding tissue, causing the tissue to compress, deform and expand. The tissue, of course, has an inherent limit of deformability. When the dilation pressure causes the tissue to deform beyond that limit, the tissue tears apart, often to form a jagged wound, with considerable damage, trauma, pain and bleeding.

U.S. Patent 4, 799, 479, and International patent Application No. WO-A-89 01800 show use of a balloon to open up an artery and then utilizes a laser, heated wire mesh, or the like, to heat up blood trapped between the media and the plaque so that dilation will be maintained and so that a smooth wall will result.

In German Patent 3,402,573 a solid core is provided with three longitudinal slots into which respective ones of three bladder segments can be retracted. Each bladder segment carries a cutting member and the opposite ends of the bladder section are anchored to the exterior of the solid core so that when the bladder segment is retracted into the slot, the cutter is also retracted into the slot. The anchoring of the bladder segment to the core is thus essential in order to ensure that the cutter remains in a position in which it can be retracted into the corresponding slot in the core.

The object of the present invention is to provide an improved dilation catheter assembly.

According to the present invention there is provided a dilation catheter assembly, comprising an elongate support having a distal end carrying a radially dilatable member adapted to be positioned longitudinally in a body conduit and having properties for dilating generally radially of the elongate support; means for dilating the dilatable member to exert dilation forces on tissue surrounding the body conduit; and a cutting element located externally of the dilatable member and carried by the support, the cutting element being movable by the dilatable member radially into contact with said tissue and operable to incise said tissue, characterised in that the dilatable member comprises a longitudinally inextensible generally cylindrical radially dilatable body operable to dilate in a generally radially symmetrical manner to a fixed volume and shape so as to exert pressure on surrounding body tissue to provide a substantially uniform tangential tension therein; thereby reducing damage to said tissue from the dilation forces.

Dilation catheter assemblies embodying the present invention will now be described by way of example with reference to the accompanying diagrammatic drawings, wherein like numbers denote like parts throughout and in which:
Figure 1 is a partly cross-sectional isometric view of one embodiment of the invention catheter;
Figure 2 is a cross-sectional view taken along line 2-2 of Figure 1;
Figure 3 is a prospective, schematic sectional view of a portion of another embodiment of the invention catheter positioned within a body conduit;
Figure 4 is a sectional, side view of the embodiment of Figure 3 in its deflated state;
Figure 5 is a sectional side view of the embodiment of Figure 3 in its inflated state;
Figure 6 is a cross-sectional view along line 6-6 of Figure 5;
Figure 7 is a sectional elevational view of another embodiment of the invention; and
Figure 8 is a view similar to Figure 1 and which includes means for selectively incising more deeply into a patient's tissue.

### Best Mode For Carrying Out Invention

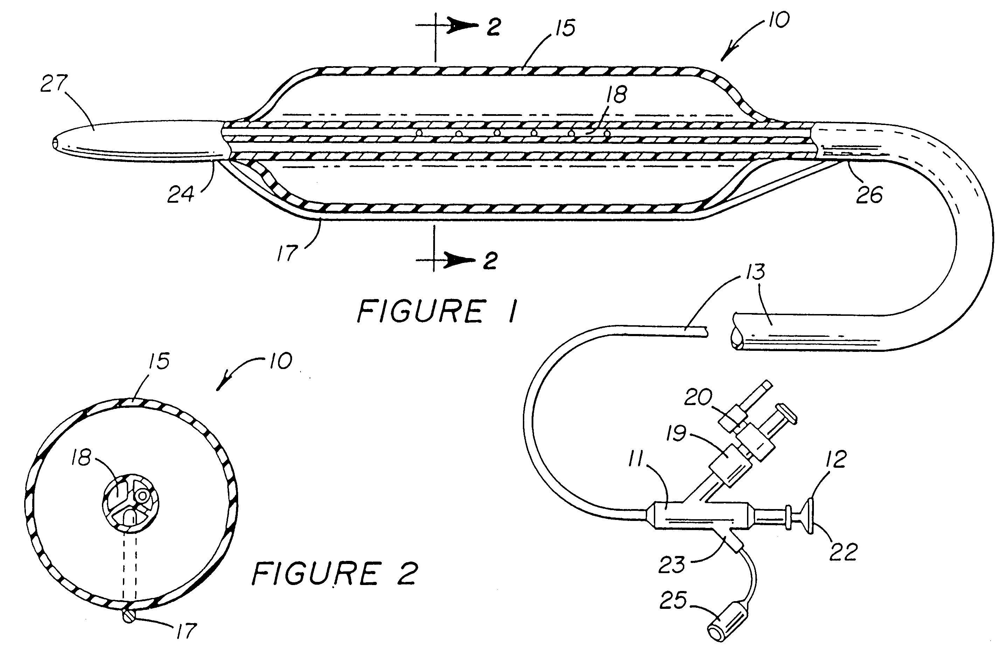

Figure 1 depicts a dilatation catheter assembly, generally designated 10, that may be used for dilating a body vessel or conduit, such as a ureter or urethra, to treat a blockage or other obstruction. The main elements of catheter assembly 10 are: an adapter 11 that defines the proximal end 12 of the assembly 10 and a site for various ports to the assembly 10; a triple lumen catheter body 13 (Figure 2); an inflatable inextensible balloon or bladder member 15; and a cutting element 17, very preferably a radio frequency cutting element activatable by a radio frequency power source. The electrosurgical cutting element or electrode 17 is in the nature of a wire which runs along generally parallel to the longitudinally extending inflatable bladder 15. In use, the bladder 15 is inserted longitudinally into a body conduit to the position where a surgical cut is required. The bladder 15 is then inflated (an inextensible bladder is used) to a desired pressure about 1 atmosphere and allowed to expand to occupy the space in the body vessel or conduit. Radio frequency current is then passed through the cutting element 17. This leads to the wire being moved outwardly and incising adjacent tissue in that direction. Thereafter, the pressurization and incising steps are suitably repeated (the pressure drops each time incising occurs) as many times as is necessary until the bladder 15 is fully inflated. In this way, the depth of the incision is controlled by the size chosen for the inextensible bladder 15 and tearing is avoided by making successive cuts rather than exerting a very large pressure within the bladder and overstressing the body conduit.

The material used for the wire can be any of the materials currently used for electrosurgical cutting wires. For example, the wire can be made of stainless steel or tungsten. A sheath with a slit in it, the slit facing away from the bladder 15, surrounds the cutting element 17. As seen in Figure 2, one of the three lumens serves as an inflation/deflation passageway 18, a second serves as a drainage/infusion passageway, and a third carries cutting element 17.

In accordance with the present invention the inflatable balloon or bladder member 15 is of the inextensible or constant volume variety, that assumes, when expanded, a specific size and shape. Thus, the balloon member 15 cannot extend significantly longitudinally within a body conduit beyond its selected length. And, further, it can only extend radially to a selected radius, thus controlling the depth of the incision. Since the balloon member 15 cannot extend longitudinally, as can elastic or elastomeric balloons, it must exert the force caused by inflation of the balloon member 15 radially against an enclosing body conduit or the like. In contrast, if an elastic or elastomeric balloon is expanded within a body conduit which has one portion particularly narrowed and particularly resistant to expansion, the balloon will simply elongate rather than acting radially outwardly against the constriction. What is happening is a balance of forces in the balloon which then expands in the direction of least resistance.

In accordance with the present invention it is preferred to utilize a radio frequency cutting element 17 for a number of reasons. One reason is that a radio frequency cutting element 17 will not perform any cutting unless and until it is activated by passing a radio frequency current through it. As a result, accidental cuts cannot be made away from the area where cutting is desired. And, with proper control cutting can be very sharply defined leading to a clean incision without tearing. This radio frequency cutting or cauterizing technique can, thus, provide significant advantages over the use of prior art cutters in an apparatus of the nature disclosed herein.

In accordance with the present invention the balloon member 15 generally extends longitudinally along the body conduit and is generally symmetrically placed and expandable therein. In this manner, as the balloon member 15 is expanded, it exerts a substantially equal tangential tension upon the tissue defining the body conduit. This results in the incision by the cutting element 17, which would generally proceed parallel to the balloon member 15, being particularly clean. In essence, the incision when made in this manner proceeds longitudinally along the body cavity and will generally not go off at an angle as might be the case if the tangential tension in the body conduit was not substantially uniform.

In accordance with the most preferred embodiment of the present invention the cutting element 17 is a radio frequency cutting element and is parallel to the bladder member 15, the bladder member 15 extends longitudinally along the body conduit and is of an inextensible (non-elastic, non-elastomeric) nature and is symmetrically placed within the body cavity so that on expansion it exerts a substantially uniform tangential tension upon the tissue defining the body cavity. This allows all of the advantages of the present invention to be realized at one and the same time.

The adapter 11 serves as a site for a bladder inflation/deflation port 19 that is attached to a source of inflation medium (not shown) for inflating the bladder member 15 or a suction source (not shown) for deflating the bladder member 15. Port 19 has a valve 20 for regulating the inflation medium or suction, as the case may be. Port 19 connects into the proximal end of an inflation/deflation passageway 18 that extends from the port 19 to the bladder member 15. The adapter 11 also serves as a site for the drainage tube inlet/outlet port 22 and a cutting element port 23. The drainage port 22 is connected to the proximal end of the lumen that carries a stylet or guide wire. The drainage port 22 may serve as a site for removing fluid from the lumen or as a site for infusing fluid into the lumen. The distal end of the catheter body has a series of drain holes 24 to facilitate flushing the lumen with fluid or voiding the bladder member 15. A "banana plug" cutting element connector 25 is affixed to the end of the cutting element port and the cutting element 17 extends from the connector through the lumen of the catheter body 13 and exits therefrom via an aperture 26 and continues along the exterior of the bladder member 15. The cutting element 17 can consist of a thin wire which has an external incising edge that faces outwardly from the bladder member 15. Alternatively, the cutting element 17 may be a sharp edge, beam, or, more preferably, a radio frequency cutting or cauterizing element 17. The element/bladder is/are constructed (e.g., the element 17 is flexible or expandable) such that the cutting element 17 is carried on the exterior of the bladder member 15 (at least when the bladder member 15 is inflated) but is not capable of incising the bladder member 15. If desired, the portion of the exterior of the bladder member 15 that is exposed to the cutting element 17 may carry a protective cover (not shown) to further guard against the bladder member 15 being incised by the cutting element 17. The cutting element 17 may be carried at a predetermined spacing from the bladder surface or directly on the surface. When carried on the surface the cutting element 17 may be an integral part of the surface or attached to the surface. If desired the cutting element 17 may be extended/retracted manually via the connector into/out of the catheter body 13.

For use in urethral dilatation the distal end of the assembly 10 includes an atromatic tip 27. Such structure may not be necessary or desirable for dilating other conduits/orifices. For urethral dilatation, the assembly 10 may optionally include another lumen and "Foley" (Registered Trade Mark) type balloon (not shown) distally of the dilatation bladder member 15 to anchor the catheter in the bladder neck of the human body to facilitate correct positioning of the dilatation bladder member 15 and minimize the possibility of migration and displacement of the assembly 10.

One or more of the catheter assembly components may be made of radiopaque materials to facilitate the visualization of the assembly 10 by the physician during placement of the assembly 10 in the body vessel/conduit.

A typical surgical procedure in which the catheter assembly 10 is employed involves the following steps. A cytoscope is first inserted into the vessel/conduit/orifice to be dilated. Calibration devices may be inserted through the cytoscope to facilitate measuring the extent of the vessel/conduit/orifice being dilated. The dilatation catheter of Figure 1 is then inserted to the desired depth in the vessel/conduit. A cytoscope lens may then be inserted to allow visualization of the catheter and the bladder location. Fluid may be infused through the drainage tube or cytoscope to facilitate such visualization. Once in position, the bladder member 15 is inflated. Such inflation causes the cutting element 17 to move radially outwardly as the bladder surface expands radially until the cutting element 17 contacts the surrounding tissue. In accordance with a preferred embodiment of the invention the bladder member 15 is inextensible as mentioned previously.

As used herein the term "tissue" is intended to include, without limitation, normal tissue, stomatic tissue, neoplastic tissue (tumors) or an obstruction such as plaque. Continued radial expansion of the bladder member 15 positions the cutting element 17 and causes the bladder member 15 to exert pressure on the tissue thereby subjecting the tissue to a substantially uniform tangential tension. If the preferred radio frequency cutting element 17 is utilized a radio frequency current is passed through it. This combined cutting and dilating action results in the tissue being expanded without being torn due to a buildup of excess stresses within the tissue. Instead, the tissue is cut in a clean, concentrated, generally longitudinal fashion by the cutting element 17 and the dilatation does not uncontrollably tear the tissue and cause excessive trauma and bleeding. The inflated bladder member 15 provides the additional benefit of acting as a tamponade to reduce bleeding. The radio frequency cutting element 17 is such that it incises the surrounding tissue in a manner such as to cause controlled incising under the combined cutting and dilating action.

After the vessel/conduit/orifice tissue is incised and dilated and the blockage/obstruction is relieved, the power through the radio frequency cutting element 17 is disconnected, if such a cutting element is used, the bladder member 15 is deflated by connecting the inflation/deflation port 19 to suction or atmospheric pressure and opening the inflation/deflation port valve 20 thereto. Deflation of the bladder member 15 results in a simultaneously radial retraction of the cutting element 17 out of contact with the tissue. Once the bladder member 15 is deflated the cutting element 17 may be retracted via the connector 25. If desired, the cutting element 17 may be retracted prior to complete deflation of the bladder member 15 and/or the bladder member 15 reinflated and left in place to act as a tamponade. Often it is desirable to leave the cutting element 17 in contact with the wound for a time, for example from about 10 minutes to about 2 hours, until the bleeding stops or is under control, and then to deflate the bladder member 15 and withdraw the catheter. Alternatively, the catheter can simply be withdrawn from the vessel/conduit altogether. The particular details of operation are determined by the surgeon depending upon the particular procedure being carried out and the particular person being operated upon.

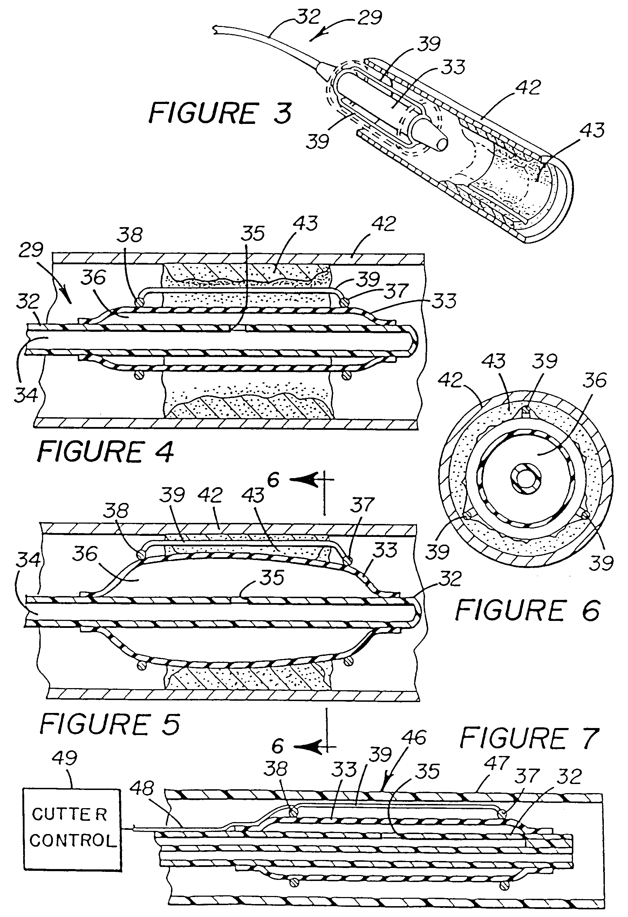

Figures 3-6 depict another dilation catheter assembly of a preferred embodiment of the invention, generally designated 29, in use. Only the distal end of the assembly 29 is shown. Adapter(s), inflation/deflation ports are not shown for convenience. The distal end is defined by a closed end catheter tube 32 which carries an inflatable, preferably inextensible, bladder member 33 on its exterior. The lumen 34 of the tube 32 is connected to the source of inflation fluid/suction, as the case may be. The tube 32 has a radial aperture 35 that opens into the lumen 36 of the bladder member 33. A pair of expandable ring-shaped members 37,38 extend around the exterior of the bladder member 33 near the distal and proximal ends thereof. One or more cutting elements 39 are affixed between the rings so that they extend longitudinally and outwardly therefrom.

Figures 3 (in solid line) and 4 show the assembly 29 in its deflated state positioned within a vessel 42 partially obstructed by an obstruction 43. In order to inflate the bladder member 33, pressurized fluid is passed through catheter tube lumen 34 and aperture 35 into the bladder lumen. Inflation of the bladder member 33 in turn causes the ring members 37,38 to expand and move the cutting element(s) 39 radially outward. Figures 3 (phantom line), 5, and 6 show the bladder member 33 in an inflated state with the cutting element 39 incising the obstruction.

Figure 7 shows yet another dilation catheter assembly, generally designated 46, of a preferred embodiment of the invention. The assembly 46 is shown in its deflated state. This assembly 46 is similar in structure to assembly 29 except that the assembly 46 is housed within a sheath or introducer 47 and a cauterizing element 48 is connected to the cutting element 39. The sheath permits the assembly 46 to be introduced into the vessel in an unexposed manner, ejected from the end thereof for use, and retracted back into the sheath 47 after use. The ejection and retraction may be achieved by relative longitudinal movement of the sheath 47, assembly 46, or both. The cutting element 39 can be a radio frequency cutting element and cauterization will result along with the cutting. Also, following cutting a coagulation producing radio frequency signal can be passed through cutting element 39.

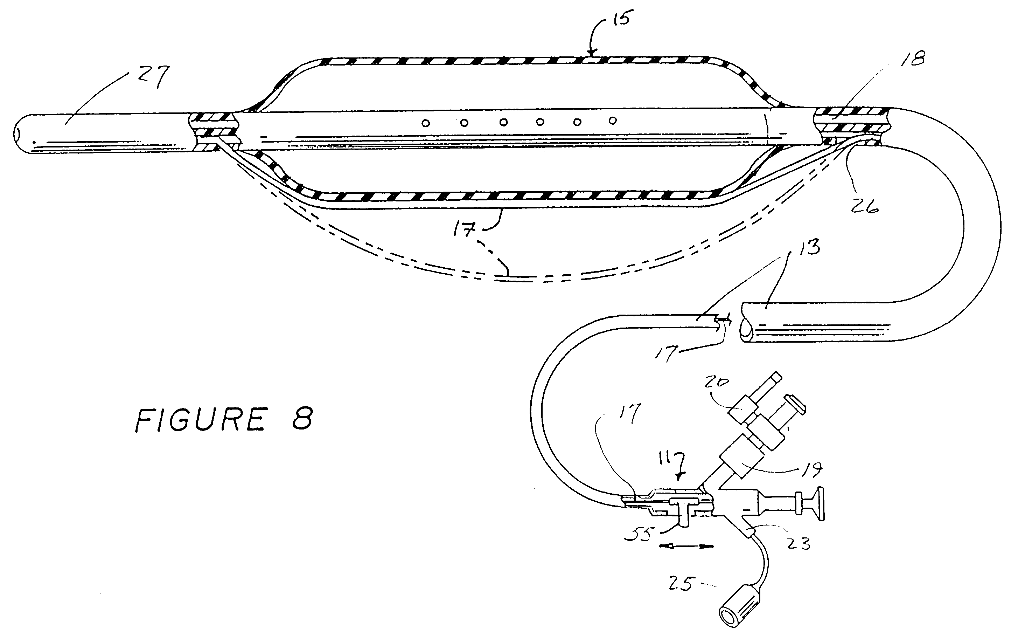

Figure 8 illustrates an embodiment of the invention wherein the cutting element 17 can be advanced beyond the supporting surface of the bladder 15. In the embodiment of Figure 8 the cutting element 17 has an actuator 55 attached to it in the area of the adapter 11, the actuator extending through the adapter 11. After incision has been completed using the bladder 15, that is, after the bladder 15 has been fully extended and the cutting element 17 has cut as deeply as it will when supported by the bladder 15, the surgeon can electively advance the actuator 55, thereby advancing the cutting element 17 and causing it to bow outwardly and away from the bladder 15, and when a radio frequency current is passed through it, incise further into the tissue a desired distance.

The described apparatus for controlled surgical dilation and incision within body conduits, vessels and orifices, virtually eliminates tearing and trauma caused by dilation beyond the strength of the tissue forming the body conduit, vessel or orifice being dilated.

## Claims

1. A dilation catheter assembly (10), comprising an elongate support (13) having a distal end carrying a radially dilatable member (15) adapted to be positioned longitudinally in a body conduit and having properties for dilating generally radially of the elongate support (13); means for dilating the dilatable member to exert dilation forces on tissue surrounding the body conduit; and a cutting element (17) located externally of the dilatable member (15) and carried by the support (13), the cutting element (17) being movable by the dilatable member (15) radially into contact with said tissue and operable to incise said tissue, characterised in that the dilatable member (15) comprises a longitudinally inextensible generally cylindrical radially dilatable body operable to dilate in a generally radially symmetrical manner to a fixed volume and shape so as to exert pressure on surrounding body tissue to provide a substantially uniform tangential tension therein; thereby reducing damage to said tissue from the dilation forces.

2. An assembly according to Claim 1 characterised in that the dilatable member (15) is an inflatable bladder.

3. An assembly according to Claim 1 or to Claim 2 characterised in that the cutting element (17), is responsive to a radio frequency electrical signal to incise said tissue.

4. An assembly according to any preceding claim characterised in that the cutting element (17) is disposed in a plane including the elongate axis of the support.

5. An assembly according to any preceding claim characterised in that the cutting element (17) is permanently affixed to the exterior surface of the dilatable member (15).

6. An assembly according to any one of Claims 1 to 4 characterised in that the cutting element (17) is removably carried on the exterior of the dilatable member (15).

7. An assembly according to any one of Claims 1 to 4 characterised in that wherein the cutting element (17) is an integral component of the dilatable member (15).

8. An assembly according to any preceding claim characterised in that the cutting element (17) comprises a thin wire.

9. An assembly according to any preceding claim characterised by an open ended sheath (42), in which the support (13) is housed, from which the support (13) may be deployed for use and into which the support (13) may be retracted after use.

10. An assembly according to any one of Claims 1 to 4 characterised in that the cutting element (17) comprises a wire disposed to extend generally longitudinally of the support (13) a first portion of the wire being rigid with the support (13) and a second portion of the wire being in a movable relationship with the support (13).

11. An assembly according to Claim 10 characterised by means disposed between the cutting element (17) and the dilatable member (15), for directing energy from the cutting element (17) away from dilatable member and toward the tissue.

12. An assembly according to Claim 11 characterised in that the directing means includes electrical insulation disposed along the cutting element (17) between the cutting element (17) and dilatable member (15).

13. An assembly according to Claim 12 characterised in that the insulation forms a sleeve around the cutting element (17) and portions of the sleeve are removed along the cutting element (17) to direct the energy toward the tissue.

14. An assembly according to any preceding claim characterised by means for maintaining a tensile force on the cutting element.

15. An assembly according to Claim 14 characterised in that portions of the assembly (10) define a lumen and a hole extending from the lumen exteriorly of the assembly; the cutting element (17) has a proximal end and a distal end; with one of said ends of the cutting element (17) extending through the hole into the lumen of the catheter.

16. An assembly according to Claim 14 or to Claim 15 characterised in that the maintaining means comprises a tension spring with a first portion having a fixed relationship with the support (13) and a second portion having a fixed relationship with the other end of the cutting element (17).

## Patentansprüche

1. Dilatationskathetereinheit (10) mit Schneideelement mit einem langgestreckten Träger (13), dessen distales Ende ein radial aufweitbares Element (15) trägt, das in Langsrichtung in eine Körperröhre eingeführt werden und sich im allgemeinen radial zum langgestreckten Träger (13) aufweiten kann, ferner mit Mitteln zum Aufweiten des aufweitbaren Elements, so daß es Dilatationskräfte auf das de Körperröhre umgebende Gewebe ausübt, und einem relativ zum aufweitbaren Element (15) außen auf dem Träger (13) angeordneten Schneideelement 17, das mittels des aufweitbaren Elements (15) radial an dem Gewebe in Anlage gebracht und derart betätigt werden kann, daß es in das Gewebe einschneidet, dadurch gekennzeichnet, daß das aufweitbare Element (15) einen in Längsrichtung nicht dehnbaren, im a gemeinen zylindrischen, radial aufweitbaren Körper umfaßt, der derart betätigt werden kann, daß er sich im wesentlichen radial symmetrisch auf ein bestimmtes Volumen und eine bestimmte Form aufweitet und damit Druck auf das umgebende Körpergewebe ausübt und darin eine im wesentlichen gleichmäßige tangentiale Spannung erzeugt, wodurch Versetzungen das Gewebes durch die Dilatationskräfte vermindert werden.

2. Einheit nach Anspruch 1, dadurch gekennzeichnet, daß das aufweitbare Element (15) eine aufweitbare Base ist.

3. Einheit nach Anspruch 1 oder 2, dadurch gekennzeichnet, daß das Schneideelement (17) auf ein elektrisches Hochfrequenzsignal hin in das Gewebe einschneidet.

4. Einheit nach einem der vorhergehenden Ansprüche, dadurch gekennzeichnet, daß das Schneideelement (17) in einer Ebene angeordnet ist, in dar auch die Längsachse das Trägers liegt.

5. Einheit nach einem der vorhergehenden Ansprüche, dadurch gekennzeichnet, daß das Schneideelement (17) dauerhaft an der Außenfläche des aufweitbaren Elements (15) befestigt ist.

6. Einheit nach einem der Ansprüche 1 bis 4, dadurch gekennzeichnet, daß das Schneideelement (17) auf der Außenfläche des aufweitbaren Elements (15) abnehmbar befestigt ist.

7. Einheit nach einem der Ansprüche 1 bis 4, dadurch gekennzeichnet, daß das Schneideelement (17) einstückig mit dem aufweltbaren Element (15) ausgebildet ist.

8. Einheit nach einem der vorhergehenden Ansprüche, dadurch gekennzeichnet daß das Schneideelement (17) einen dünnen Draht umfaßt.

9. Einheit nach einem der vorhergehenden Ansprüche, gekennzeichnet durch einen Mantel (42) mit offenem Ende, in dem der Träger (13) untergebracht ist und aus dem der Träger (13) zum Gebrauch vorgeschoben und in den er nach Gebrauch wieder zurückgezogen werden kann.

10. Einheit nach einem der Ansprüche 1 bis 4, dadurch gekennzeichnet, daß das Schneideelement (17) einen sich im wesentlichen in Längsrichtung des Trägers (13) erstreckenden Draht aufweist, wobei ein erster Teil des Drahtes starr mit dem Träger (13) verbinden ist und ein zweiter Teil des Drahtes gegenüber dem Träger (13) beweglich ist.

11. Einheit nach Anspruch 10, gekennzeichnet durch zwischen dem Schneideelement 17 und dem aufweitbaren Element 15 angeordnete Mittel, mittels derer Energie vom Schneideelement (17) in Richtung vom aufweitbaren Element weg und zum Gewebe hin geleitet werden kann.

12. Einheit nach Anspruch 11, dadurch gekennzeichnet, daß die Energieleitmittel eine entlang das Schneideelements (17) zwischen dem Schneideelement (17) und dem aufweitbaren Element (15) angebrachte elektrische Isolierung aufweisen.

13. Einheit nach Anspruch 12, dadurch gekennzeichnet, daß die Isolierung eine Hülse um das Schneideelement (17) ausbildet und daß Teile der Hülse entlang des Schneideelements (17) entfernt sind, um die Energie auf das Gewebe aufzubringen.

14. Einheit nach einem dar vorhergehenden Ansprüche, gekennzeichnet durch Mittel zum Aufrechterhalten einer auf das Schneideelement wirkenden Zugkraft.

15. Einheit nach Anspruch 14, dadurch gekennzeichnet, daß Teile der Einheit (10) ein Lumen und eine sich vom Lumen relativ zur Einheit auswärts erstreckende Öffnung ausbilden und daß das Schneideelement (17) ein proximales und ein distales Ende aufweist, wobei eines der Enden des Schneideelements (17) sich durch die Öffnung in das Lumen des Katheters erstreckt.

16. Einheit nach Anspruch 14 oder 15, dadurch gekennzeichnet, daß die Mittel zum Aufrechterhalten der Spannung eine Zugfeder mit einem ersten, in fester Beziehung zum Träger (13) stehenden Teil und einem zweiten, in fester Beziehung zum anderen Ende des Schneideelements (17) stehenden Teil umfassen.

## Revendications

1. Ensemble de cathéter (10) de dilatation, comprenant un support allongé (13) qui comporte une extrémité distale portant un organe dilatable radialement (15) apte à être positionné longitudinalement dans un conduit corporel et qui possède des propriétés lui permettant de se dilater d'une manière généralement radiale par rapport au support allongé (13); un moyen de dilatation de l'organe dilatable pour exercer des forces de dilatation sur le tissu qui entoure le conduit corporel; et un élément coupant (17) situé à l'extérieur de l'organe dilatable (15) et porté par le support (13), ledit élément coupant (17) étant mobile radialement sous l'effet de l'élément dilatable (15) pour venir au contact dudit tissu et pouvant intervenir pour inciser ledit tissu, caractérisé en ce que l'organe dilatable (15) comprend un corps de forme générale Cylindrique, dilatable radialement et inextensible longitudinalement, qui peut être mis en oeuvre pour se dilater d'une manière généralement symétrique radialement pour prendre un volume et une forme fixes de manière à exercer une pression sur le tissu corporel environnant pour y induire une tension tangentielle sensiblement uniforme en réduisant ainsi les dommages causés audit tissu par les forces de dilatation.

2. Ensemble selon la revendication 1, caractérisé en ce que l'organe dilatable (15) est une vessie gonflable.

3. Ensemble selon la revendication 1 ou la revendication 2, caractérisé en ce que l'élément coupant (17) répond à un signal électrique de radiofréquence pour inciser ledit tissu.

4. Ensemble selon l'une quelconque des revendications précédentes, caractérisé en ce que l'élément coupant (17) est disposé dans un plan qui inclut l'axe allongé du support.

5. Ensemble selon l'une quelconque des revendications précédentes, caractérisé en ce que l'élément coupant (17) est fixé en permanence à la surface extérieure de l'organe dilatable (15).

6. Ensemble selon l'une quelconque des revendications 1 à 4, caractérisé en ce que l'élément coupant (17) est porté de façon amovible sur l'extérieur de l'organe dilatable (15).

7. Ensemble selon l'une quelconque des revendications 1 à 4, caractérisé en ce que l'élément coupant (17) est un composant d'une seule pièce avec l'organe dilatable (15).

8. Ensemble selon l'une quelconque des revendications précédentes, caractérisé en ce que l'élément coupant (17) comprend un fil mince.

9. Ensemble selon l'une quelconque des revendications précédentes, caractérisé par un fourreau (42) à extrémités ouvertes, dans lequel le support (13) est logé, à partir duquel le support (13) peut être déployé pour être utilisé et dans lequel le support (13) peut être rétracté après utilisation.

10. Ensemble selon l'une quelconque des revendications 1 à 4, caractérisé en ce que l'élément coupant (17) comprend un fil disposé de manière à s'étendre d'une manière généralement longitudinale par rapport au support (13), une première partie dudit fil étant rigide avec le support (13) et une deuxième partie du fil étant en relation de mobilité par rapport audit support (13).

11. Ensemble selon la revendication 10, caractérisé par un moyen disposé entre l'élément coupant (17) et l'organe dilatable (15), pour diriger en s'écartant de l'organe dilatable et vers le tissu, une énergie venant de l'élément coupant (17).

12. Ensemble selon la revendication 11, caractérisé en ce que le moyen directionnel inclut un isolant électrique disposé le long de l'élément coupant (17), entre l'élément coupant (17) et l'organe dilatable (15).

13. Ensemble selon la revendication 12, caractérisé en ce que l'isolant forme un manchon autour de l'élément Coupant (17), et des parties du manchon sont enlevées le long de l'élément Coupant (17) pour diriger l'énergie vers le tissu.

14. Ensemble selon l'une quelconque des revendications précédentes, caractérisé par un moyen de maintien d'une force de tension sur l'élément coupant.

15. Ensemble selon la revendication 14, caractérisé en ce que des parties de l'ensemble (10) définissent un passage et un orifice s'étendant à partir dudit passage à l'extérieur de l'ensemble; l'élément coupant (17) comporte une extrémité proximale et une extrémité distale; et l'une desdites extrémités de l'élément coupant (17) s'étend à travers l'orifice pour entrer dans le passage du cathéter.

16. Ensemble selon la revendication 14 ou la revendication 15, caractérisé en ce que le moyen de maintien comprend un ressort de tension dont une première partie est en relation fixe avec le support (13) et une deuxième partie est en relation fixe avec l'autre extrémité de l'élément coupant (17).
